# EUROPÄISCHE PATENTSCHRIFT

(11) **EP 0 811 846 B1**
(45) Veröffentlichungstag und Bekanntmachung des Hinweises auf die Patenterteilung: **16.10.2002**
(21) Anmeldenummer: 97109117.8
(22) Anmeldetag: 05.06.1997
(51) Int. Cl.: G01N 33/553, C12N 9/96, C12N 11/14, G01N 33/58

(54) **Stabilisierung von Metallkonjugaten**
Stabilisation of metal conjugates
Stabilisation de conjugués métalliques

(30) Priorität: 05.06.1996 DE 19622628
(43) Veröffentlichungstag der Anmeldung: 10.12.1997
(73) Patentinhaber: Roche Diagnostics GmbH, 68305 Mannheim (DE)
(72) Erfinder: Nichtl, Alfons, Dr., 82383 Hohenpeissenberg (DE); Sluka, Peter, Dr., 82362 Weilheim (DE)
(74) Vertreter: Weiss, Wolfgang, Dipl.-Chem. Dr.

(56) Entgegenhaltungen:
- EP-A- 0 441 120
- EP-A- 0 489 465
- WO-A-90/05303
- WO-A-93/21528
- WO-A-94/21240
- US-A- 4 313 734
- C. PALE-GROSDEMANGE, E. S. SIMON, K. L. PRIME, G. M. WHITESIDES: "Formation of self-assembled monolayers by chemisorption of derivatives of oligo(ethylene glycol) of structure HS(CH2)11(OCH2CH2)mOH on gold" JOURNAL OF THE AMERICAN CHEMICAL SOCIETY, Bd. 113, Nr. 1, 1991, Seiten 12-20, XP002063516
- K. L. PRIME, G. M. WHITESIDES: "Self-assembled organic monolayers: Model systems for studying adsorption of proteins at surfaces" SCIENCE, Bd. 252, 24.Mai 1991, Seiten 1164-1167, XP002063517
- BEHNKE O; ET AL: "NON-SPECIFIC BINDING OF PROTEIN-STABILIZED GOLD SOLS AS A SOURCE OFERROR IN IMMUNOCYTOCHEMISTRY", EUROPEAN JOURNAL OF CELL BIOLOGY, WISSENSCHAFLICHE VERLAGSGESELLSCHAFT, STUTTGART, DE, August 1986, Band 41, Nr. 2, Seiten 326 - 338

## Beschreibung

Die vorliegende Erfindung betrifft Zusammensetzungen, welche eine Suspension von kolloidalen Partikeln in einer wäßrigen Lösung umfassen, wobei an der Oberfläche der Partikel Biomoleküle adsorbiert sind.

Konjugate, die aus Biomolekülen wie etwa Proteinen oder Nucleinsäuren und kolloidalen Partikeln bestehen, werden weitverbreitet in diagnostischen und therapeutischen Verfahren, z.B. als Marker in Nachweisverfahren wie etwa Immunoassays, oder als Mikroprojektile zum Gentransfer eingesetzt. Als Partikel können Partikel aus Metallen und Metallverbindungen wie etwa Metalloxiden, Metallhydroxiden, Metallsalzen und Polymerkernen, die mit Metallen oder Metallverbindungen beschichtet sind, verwendet werden (vgl. z.B. US-A-4,313,734; Leuvering et al., J.Immunoassay 1 (1980), 77-91; Leuvering Dissertation (1984), Sol Particle Immunoassay (SPIA): The Use of Antibody Coated Particles as Labelled Antibodies in Various Types of Immunoassay; Uda et al., Anal.Biochem. 218 (1994), 259-264, DE-OS 41 32 133, Seite 3, Zeilen 16-18, für Anwendungen als Marker und Tang et al., Nature 356 (1992), 152-154; Eisenbraun et al., DNA and Cell Biology 12 (1993), 791-797; Williams et al., Proc.Natl.Acad.Sci. USA 88 (1991), 2726-2730 für Anwendungen zum Gentransfer). Weiterhin ist auch die Verwendung von nichtmetallischen kolloidalen Partikeln wie etwa Carbonteilchen bekannt (van Amerongen, Anabiotic '92 (1993), 193-199). Am häufigsten werden zur Zeit kolloidale Goldpartikel eingesetzt.

Zur Herstellung von Biomolekül-Gold-Konjugaten werden zunächst nach allgemein bekannten Verfahren Goldsole durch Reduktion von Tetrachlorgoldsäure hergestellt. Anschließend erfolgt eine Beladung der Goldsole mit dem jeweils gewünschten Biomolekül, z. B. Proteinen wie Antikörpern, Protein A, Protein G, Streptavidin, etc. Die jeweiligen Beladebedingungen (pH, Puffer, Konzentration der Biomoleküle etc.) richten sich nach dem isoelektrischen Punkt des Biomoleküls, der MPA (minimal protecting amount) oder/und der spezifischen Anwendung des Konjugats (vgl. z. B. De Mey, The Preparation and Use of Gold Probes, in: Immunocytochemistry, eds: J. M. Polak und S. v. Noorden, pp 115-145, Wright, Bristol 1986 und J. E. Beesley, Colloidal Gold: A New Perspective for Cytochemical Marking, Microscopy Handbooks 17, Oxford University Press, 1989, insbesondere pp 1-14). Auf die Offenbarung dieser Dokumente wird ausdrücklich Bezug genommen.

Nach Beladung der kolloidalen Partikel mit dem jeweils gewünschten Biomolekül ist eine Stabilisierung der Konjugate notwendig. Durch diese Stabilisierung soll eine Aggregation der Partikel minimiert werden und verbleibende freie, der Adsorption zugängliche Oberflächen abgesättigt werden. Im Stand der Technik werden als Stabilisatoren inerte Proteine, z. B. Rinderserumalbumin, Blutersatzmischungen etc., Detergenzien, wie etwa Tween® 20, wasserlösliche technische Polymere wie Polyethylenglykol (Molekulargewicht 20.000 D), Polyvinylpyrrolidon, Polyvinylalkohol, Polyvinylsulfat, Dextran und Gelatine verwendet (vgl. z. B. De Mey, Supra; Beesley, Supra; Behnke, Eur. J. Cell Biol. 41 (1986), 326-338; DE 24 20 531 C3; und Meisel et al., J. Phys. Chem. 85 (1981), 179-187). Weiterhin wurde auch die Möglichkeit der Stabilisierung von Goldsolen durch Phosphan-Komplexliganden beschrieben (Schmid et al., Z. Naturforsch. 45b (1994), 989-994). EP-A-489465 offenbart die Stabilisierung von Goldsol-Partikeln durch den Zusatz verschiedener Alkanthiole sowie deren Derivate.

Die nach dem Stand der Technik eingesetzten Stabilisatoren binden adsorptiv an die freien Oberflächen der Metallpartikel. Durch längere Lagerung oder durch Änderung der Umgebungsbedingungen, wie sie z. B. in einem Test durch Kontakt mit Probe (Blut, Serum, Plasma, Urin), Einbringen der Konjugate in Teststreifenvliese etc. auftreten, können die Stabilisatoren mehr oder weniger stark von den Oberflächen desorbieren oder verdrängt werden. Dies führt zu einer Verschlechterung der Aggregationsstabilität und zu einer Zunahme der unspezifischen Reaktivität. Darüber hinaus handelt es sich bei einem großen Teil der verwendeten Stabilisatoren um schlecht definierte Produkte mit zum Teil schwankender Qualität, z. B. Rinderserumalbumin, Gelatine. Dadurch können auch Schwankungen in der Stabilisierungswirkung auftreten.

Adsorptionsvorgänge an Partikeloberflächen sind sehr komplex und bisher nur zum Teil verstanden. Es wird angenommen, daß die Adsorption über eine Kombination von elektrostatischen Wechselwirkungen, Van-der-Waals-Kräften und hydrophoben Wechselwirkungen zustande kommt (Beesley, Supra). Je nach Art des adsorbierten Biomoleküls kann dabei die eine oder die andere Bindungsart überwiegen.

Eine Veröffentlichung von Prime und Whitesides (Science 252 (1991), 1154-1167) offenbart, daß Mischungen von hydrophoben (Methyl-terminierten) und hydrophilen (Hydroxyl-, Maltose- und Hexaethylenglykol-terminierten) Alkanthiolen die Adsorption von Proteinen an planare Goldoberflächen unterdrücken können. Eine Stabilisierung der Proteinadsorption durch Alkanthiole wird weder offenbart noch nahegelegt.

Überraschenderweise wurde nun festgestellt, daß durch Thioloder/und Disulfidgruppen substituierte Polyethylenglykole sich hervorragend zur Stabilisierung von Biomolekül-Partikel-Konjugaten eignen. Durch die im Vergleich zu den Stabilisatoren des Standes der Technik stärkere Bindung des substituierten Polyethylenglykole an die Partikeloberfläche wird eine wesentliche Verbesserung hinsichtlich der Stabilität der Konjugate erreicht. Dies führt zu einer verbesserten Langzeitstabilität und einer geringeren Aggregationsneigung in Lösung, zu einer besseren Stabilität gegenüber Veränderungen der Umgebungsbedingungen und einer verbesserten Testfunktion, z. B. Chromatographieeigenschaften. Besonders überraschend war, daß diese Verbesserungen erreicht werden konnten, ohne daß dabei die Funktion der Konjugate, beispielsweise durch Verdrängung der Biomoleküle durch das substituierte Polyethylenglykol oder durch mögliche Wechselwirkungen der Biomoleküle mit dem substituierten Polyethylenglykol (z. B. Wechselwirkungen zwischen SH-Gruppen der Biomoleküle und SH-Gruppen der substituierten Polyethylenglykols) im Test negativ beeinflußt wurde. Im Gegenteil findet man sogar bei vielen Testformaten durch den Einsatz der substituierten Polyethylenglykole eine verbesserte Testperformance, die sich in einer Verringerung der unteren Nachweisgrenze oder/und einer höheren Dynamik (steilere Nachweiskurve) zeigen kann. Geeignet als Stabilisator sind dabei kürzerkettige, definierte substituierte Polyethylenglykole mit beispielsweise mindestens 2 Ethylenoxideinheiten, höhermolekulare substituierte Polyethylenglykole mit einer statistischen Molmassenverteilung und Mischungen davon.

Ein Gegenstand der vorliegenden Erfindung ist somit eine Zusammensetzung, umfassend kolloidale Partikel, an deren Oberfläche Biomoleküle adsorbiert sind, wobei die Zusammensetzung weiterhin ein durch Thiol- oder/und Disulfidgruppen substituiertes Polyethylenglykol enthält, welches vorzugsweise an der Oberfläche der Partikel coadsorbiert ist.

Die erfindungsgemäße Zusammensetzung kann als wäßrige Suspension oder auch immobilisiert, beispielsweise auf einem Chromatographiematerial, wie etwa einem saugfähigen Papier, vorliegen. Wenn die Zusammensetzung in Form einer wäßrigen Suspension ist, enthält sie vorzugsweise neben dem an der Oberfläche der Partikel coadsorbierten substituierten Polyethylenglykol dieses zusätzlich im Puffer in gelöster Form.

Die Partikel können metallische oder nichtmetallische Partikel wie etwa Carbonpartikel sein. Bevorzugt sind metallische Partikel wie etwa Partikel aus Metallen, Metalloxiden, Metallhydroxiden, Metallverbindungen, oder mit Metallen oder Metallverbindungen beschichtete Partikel. Besonders bevorzugt sind Metallpartikel.

Die Metallpartikel sind vorzugsweise Edelmetallpartikel, z. B. Metalle, ausgewählt aus der Gruppe bestehend aus Gold, Silber, Kupfer, Platin, Palladium und Mischungen davon. Besonders bevorzugt sind Goldpartikel.

Der mittlere Durchmesser der Partikel liegt - wie im Stand der Technik üblich - im Bereich von 1-1000 nm und kann je nach Anwendungszweck variiert werden. Bevorzugt ist der mittlere Durchmesser der Partikel im Bereich von 2-200 nm und besonders bevorzugt von 2-100 nm.

Die an der Oberfläche der Partikel adsorbierten Biomoleküle sind vorzugsweise ausgewählt aus der Gruppe bestehend aus Proteinen, Glycoproteinen, Peptiden, Nucleinsäuren, peptidischen Nucleinsäuren, Sacchariden, Antigenen und Haptenen. Besonders bevorzugt werden Biomoleküle ausgewählt aus der Gruppe bestehend aus Antikörpern, Antikörperfragmenten, Lectinen, Enzymen, Streptavidin, Avidin, Protein A, Antigenen wie etwa rekombinanten Polypeptiden oder multiplen Antigenen (vgl. WO96/03652), z.B. Polyhaptenen (mehrere an einen Träger wie etwa Dextran oder ein Protein gekoppelte Haptene oder Peptide), Peptiden und Haptenen (niedermolekulare Substanzen vorzugsweise mit einem MW ≤ 1500 wie etwa Biotin, Fluorescein oder Digoxigenin). Bezüglich der genauen Bedingungen zur Adsorption dieser Biomoleküle an Goldpartikel wird auf die bereits genannten Übersichtsartikel De Mey und Beesley verwiesen.

Ein als Stabilisator geeignetes Thiol-substituiertes Polyethylenglykol ist vorzugsweise ein im wesentlichen lineares kettenförmiges Molekül, das an mindestens einem Ende der Kette eine Thiolgruppe trägt. Vorzugsweise ist an einem Ende eine Thiolgruppe und am anderen Ende eine von Thiol oder Disulfid verschiedene Gruppe. Ein als Stabilisator geeignetes Disulfid-substituiertes Polyethylenglykol ist vorzugsweise ebenfalls ein im wesentlichen lineares kettenförmiges Molekül, das innerhalb der Kette mindestens eine SS-Brücke enthält. Bevorzugt enthält das Molekül nur eine SS-Brücke und an den Enden von Thiol oder Disulfid verschiedene Gruppen. Auch Mischungen von Disulfid- und Thiol-substituierten Verbindungen sind als Stabilisator geeignet.

Das durch Thiol- oder/und Disulfidgruppen substituiertes Polyethylenglykol weist somit vorzugsweise die allgemeine Strukturformel auf:

HS - R¹ (I)

oder 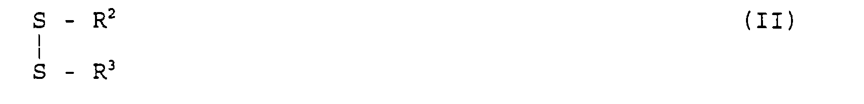 worin der Rest R¹ sowie mindestens einer der Reste R² und R³ ein organischer Rest ist, der mindestens 2 und vorzugsweise mindestens 3 (CH₂-CH₂-O)-Gruppen enthält.

Die Ethylenoxideinheiten enthaltenden Reste R₁, R₂ und R₃ der substituierten Polyethylenglykole werden vorzugsweise so gewählt, daß die Verbindungen ein maximales mittleres Molekulargewicht von etwa 50.000 D und besonders bevorzugt ein maximales mittleres Molekulargewicht von etwa 25.000 D aufweisen. Die Endgruppen der Reste R₁, R₂ und R₃ können an sich beliebig sein, bevorzugt sind jedoch OH-Gruppen oder/und O-Alkylgruppen, z. B. O-C₁-C₄-Alkylgruppen, wie etwa O-Methyl- oder O-Ethylgruppen.

Ein bevorzugtes Beispiel für ein geeignetes substituiertes Polyethylenglykol ist ein kommerziell von Shearwater Polymers Inc. erhältliches Methoxy-Polyethylenglykol-SH mit einem mittleren Molekulargewicht von ca. 5.000 D. Andererseits können auch kurze definierte Thiol- oder Disulfid-substituierte Polyethylenglykole verwendet werden, die beispielsweise 2-10 Ethylenoxideinheiten enthalten.

Die erfindungsgemäßen Zusammensetzungen können als Nachweisreagenz, insbesondere als immunologisches Nachweisreagenz verwendet werden. In einer ersten bevorzugten Ausführungsform wird das Nachweisreagenz in einem Immunoassay eingesetzt, d. h. in einem Verfahren zur Bestimmung eines Analyten in einer Probeflüssigkeit mittels immunologischer Methoden, z.B. durch einen kompetitiven Assay, bei dem ein markiertes Analytenanolog oder ein markierter, Analyt-spezifischer Rezeptor, z.B. ein Antikörper eingesetzt wird, oder einen Sandwich-Assay, bei dem ein markierter Analyt-spezifischer Rezeptor oder ein markierter, mit dem Analyt-spezifischen Rezeptor bindefähiger weiterer Rezeptor eingesetzt wird. Bevorzugte Beispiele sind Schwangerschaftstests, z. B. Tests zum Nachweis von Humanchoriongonadotropin (HCG), oder Verfahren zum Nachweis von Drogen wie etwa Kokain oder Amphetaminen, Humanserumalbumin, Troponin T, Myoglobin und Immunglobulinen wie etwa Anti-HIV-Antikörpern. Besonders bevorzugte Anwendungsformen sind Schnelltests, bei denen die zu bestimmende Probe auf ein saugfähiges, das Nachweisreagenz enthaltende Material, z.B. einen Teststreifen, gegeben wird. Eine zweite besonders bevorzugte Ausführungsform, bei der die erfindungsgemäß stabilisierte Zusammensetzung verwendet werden kann, ist die Anfärbung von Gewebeschnitten.

Außerdem können die erfindungsgemäßen Zusammensetzungen natürlich auch für alle weiteren Anwendungen eingesetzt werden, die für Biomolekül-Partikel-Konjugate bekannt sind, z.B. zum Gentransfer.

Noch ein weiterer Gegenstand der vorliegenden Erfindung ist ein Verfahren zur Stabilisierung von Konjugaten aus kolloidalen Partikeln und Biomolekülen, wobei man dem Konjugat ein durch Thiol- oder/und Disulfidgruppen-substituiertes Polyethylenglykol zusetzt. Auf diese Weise kann eine erhöhte Langzeitstabilität der Konjugate (> 1,5 Jahre) sowie eine verbesserte pH-Stabilität und eine verbesserte Stabilität gegenüber der Anwesenheit anderer Substanzen erreicht werden.

Vorzugsweise wird das substituierte Polyethylenglykol in einer Menge zugesetzt, um eine Endkonzentration von 0,1 µM bis 1 mM, insbesondere von 1 µM bis 100 µM zu ergeben.

Weiterhin kann man zusätzliche, aus dem Stand der Technik bekannte Stabilisatoren, wie etwa inerte Proteine, z. B. Rinderserumalbumin, oder/und unsubstituierte Polyethylenglykole einsetzen.

Schließlich betrifft die vorliegende Erfindung auch einen Testkit für ein immunologisches Nachweisverfahren, der eine erfindungsgemäß stabilisierte Zusammensetzung als Nachweisreagenz enthält.

Weiterhin soll die Erfindung durch die Abbildung und die nachfolgenden Beispiele erläutert werden.

Es zeigt:

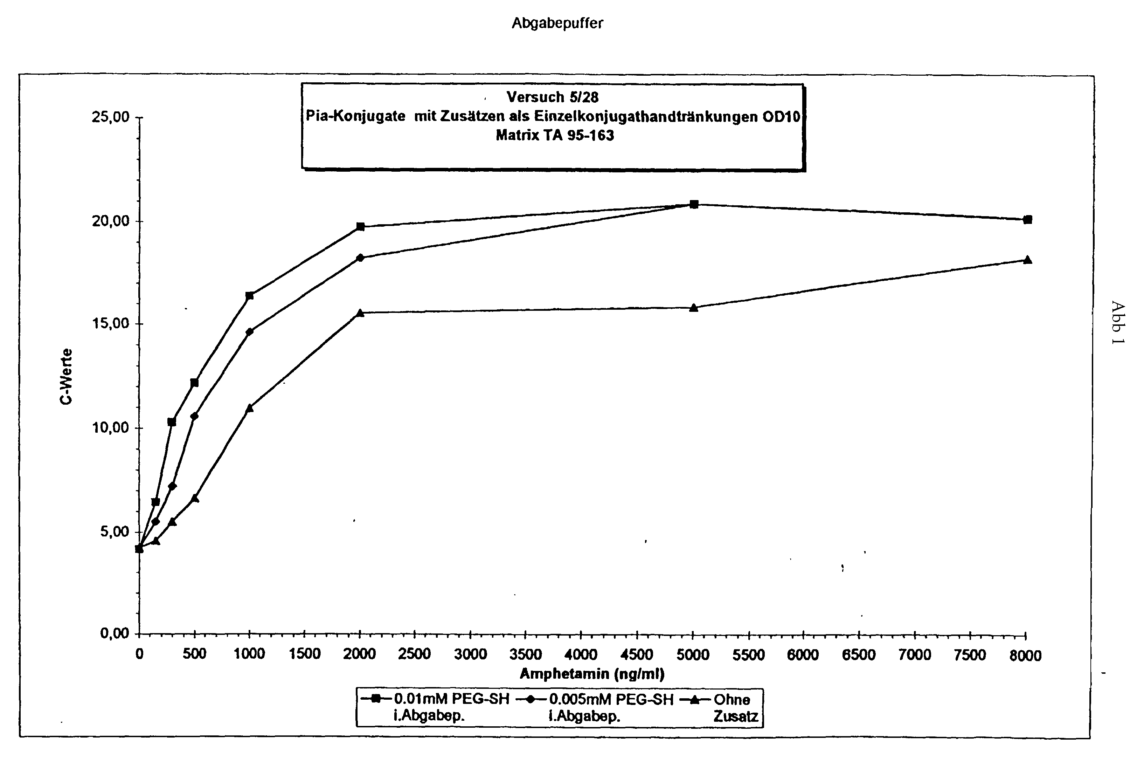

- Abb. 1: einen Vergleich der Testperformance von erfindungsgemäß stabilisierten Antikörper-Gold-Konjugaten mit Konjugaten des Standes der Technik.

### Beispiel 1

### Synthese von Thiol-substituierten Polyethylenglykol-Verbindungen

25 g Tetraethylenglykolmonomethylether (Fa. Kodak) wurden mit 25 g Tosylchlorid in 150 ml Pyridin umgesetzt. Der Ansatz wurde mit Wasser verdünnt und anschließend mit Essigester extrahiert. Das durch Einengen gewonnene Tosylat (13 g) wurde mit 15 g Phthalimid-Kaliumsalz in DMF bei 150 °C umgesetzt. Das Reaktionsgemisch wurde mit Wasser verdünnt und mit Essigester extrahiert. Das nach Einrotieren erhaltene Produkt (10,2 g) wurde in 50 ml Methanol aufgenommen, filtriert und mit 1,83 ml Hydrazinhydrat versetzt. Der Ansatz wurde 2 Stunden unter Rückfluß gekocht, abgekühlt, mit konzentrierter HCl versetzt und vom ausgefallenen Niederschlag abfiltriert.

Das Filtrat wurde mit 3 N NaOH alkalisch gemacht und anschließend mit Chloroform extrahiert. Nach Einrotieren wurden 3,11 g eines viskosen Öls erhalten.

500 mg dieses Öls wurden mit 244 mg Triethylamin in 10 ml THF gelöst. Zu dieser Lösung wurden 591 mg S-Acetylthiopropionsäure-O-succinimidester in 20 ml THF zugetropft. Diese Lösung wurde 24 h bei Raumtemperatur gerührt, einrotier und über eine Kieselgelsäule aufgereinigt.

Zur Abspaltung der Acetylgruppe, bei der die Thiolgruppen freigesetzt werden, wurden 15 ml 25-%ige Ammoniaklösung zu einer Lösung von 550 mg des Produktes in Wasser gegeben. Die Aufreinigung des Ansatzes erfolgte säulenchromatographisch. Es wurden 300 mg einer Monomethoxytetraethylenglykolpropionamido-SH-Verbindung erhalten.

### Beispiel 2

### Herstellung von Protein-Goldkonjugaten

Eine Lösung des an Goldpartikel zu adsorbierenden Proteins wurde gegen einen geeigneten Beladepuffer dialysiert oder mit dem Beladepuffer verdünnt. Anschließend wurden eventuell gebildete Aggregate durch Zentrifugation oder Filtration durch einen 0,2 µm-Filter entfernt.

Der pH-Wert der die kolloidalen Goldpartikel enthaltenden Lösung wurde mit K₂CO₃ auf den pH-Wert der Proteinlösung eingestellt. Dann wurde die Proteinlösung unter Rühren der kolloidalen Goldlösung zugesetzt.

Die Stabilisierung der auf diese Weise hergestellten Protein-Gold-Konjugate gemäß dem Stand der Technik erfolgte durch Zugabe einer RSA-Lösung bis zu einer Endkonzentration von 0,1 %-1 % (w/v) oder durch Zugabe einer Polyethylenglykollösung bis zu einer Endkonzentration von 0,01 %-0,1 % (w/v).

Anschließend erfolgte eine Reinigung und Aufkonzentrierung der Protein-Gold-Konjugate sowie die Einstellung der gewünschten Lagerpufferbedingungen, z. B. 20 mM Tris, 100 mM NaCl pH 8, 1 % RSA oder/und 0,01 %-0,1 % PEG, NaN₃.

### Beispiel 3

### Vorabsättigung des Goldsols durch PEG-SH im Vergleich zu PEG

Bei Vorabsättigung des Goldsoles gemäß Beispiel 2 durch Zugabe von 10⁻⁵ M PEG-SH (kommerzielles PEG-SH MW 5000 von Shearwater Polymers, Inc. oder PEG-SH gemäß Beispiel 1) bzw. PEG vor der Zugabe eines monoklonalen Anti-Humanserumalbumin-Maus-IgG-Antikörpers zu 20 nm Goldpartikeln wurde gefunden, daß PEG durch den Antikörper vom Goldsol verdrängt werden kann, PEG-SH jedoch nicht.

### Beispiel 4

### Einsatz von erfindungsgemäß stabilisierten Protein-Gold-Konjugaten in Tests zum Nachweis von Humanserumalbumin und HCG

Gemäß der in Beispiel 2 beschriebenen Prozedur wurden Konjugate von monoklonalen Anti-Humanserumalbumin- und Anti-HCG-Antikörpern an Goldpartikel hergestellt. Nach Bildung der Konjugate erfolgte eine Stabilisierung durch Zusatz von PEG-SH in einer Endkonzentration von 10⁻⁶ M bis 10⁻⁴ M.

Es wurden die Zugabe von PEG-SH als alleinigen Stabilisator direkt nach der Antikörperbeladung, die Zugabe von PEG-SH mit RSA (nacheinander oder in Mischung) als Stabilisator direkt nach der Antikörperzugabe und die Zugabe von PEG-SH als Stabilisator zum Lagerpuffer (Endkonzentration 10⁻⁴ M und 10⁻⁵ M) getestet.

Die Tests wurden als kompetitive Ein-Schritt-Tests auf einem Teststreifen durchgeführt.

Es wurde gefunden, daß die Funktion von Konjugaten aus monoklonalen Anti-HSA-Antikörpern und Goldpartikeln durch die Stabilisierung mit PEG-SH nicht negativ beeinflußt wird.

### Beispiel 5

### Stabilität von PEG-SH-stabilisierten Goldkonjugaten

Eine Bestimmung der in Beispiel 4 hergestellten Antikörper-Gold-Konjugate nach einer Lagerzeit von 1,5 Jahren ergab, daß keine Antikörper abbluten und daß die Funktion noch vollständig vorhanden ist.

### Beispiel 6

### pH-Stabilität von Antikörper-Gold-Konjugaten

Gemäß der in Beispiel 2 beschriebenen Prozedur wurden monoklonale Anti-HCG-Maus-IgG-Antikörper an 40 nm Goldpartikel adsorbiert. Anschließend erfolgte eine Nachsättigung wie in der Erläuterung zu den Tabellen 1a, 1b und 1c beschrieben.

Es wurde die Stabilität dieser Konjugate in einem Tränkpuffer (50 mM HEPES pH 7,0, 100 mM NaCl, 2,0 % Saccharose, 0,1 % RSA, 0,2 % Brij 35) getestet, der mit HCl bzw. NaOH auf unterschiedliche pH-Werte eingestellt worden war.

Testdurchführung: Von allen getesteten Goldkonjugaten wurde die tatsächliche OD bei 520 nm gemessen. Dann wurde jede Charge mit jeweils 5 Tränkpuffern von unterschiedlichen pH-Werten auf eine OD von ca. 8 verdünnt und sofort, nach 1 und 6 Stunden am Photometer überprüft.

Tabelle 1a zeigt die Ergebnisse für ein erfindungsgemäßes Antikörper-Gold-Konjugat, das durch PEG 20.000 ad 0,05 % und anschließend PEG-SH ad 1 µM stabilisiert wurde.

Tabelle 1b zeigt die Ergebnisse für ein weiteres erfindungsgemäßes Antikörper-Goldkonjugat, das durch PEG-SH ad 1 µM stabilisiert wurde.

Tabelle 1c zeigt die Ergebnisse für ein Antikörper-Goldkonjugat des Standes der Technik, das durch Absättigung ad 0,05 % PEG stabilisiert wurde.

**Tabelle 1a**

| pH-Wert der Konjugat-Stammlsg | pH-Wert des Tränkpuffers | pH-Wert nach Verd. auf OD8 | OD der Konjugat-Stammlsg. | OD sofort n. Verdünnung | OD nach 1 Std. | OD nach 6 Std. |
|---|---|---|---|---|---|---|
| 8.5 | 4.3 | 6.7 | 19.97 | 8.3 | 8.2 | 8.4 |
| | 5.3 | 7.1 | | 8.3 | 8.2 | 8.4 |
| | 7.0 | 7.6 | | 8.3 | 8.3 | 8.5 |
| | 7.5 | 8.2 | | 8.2 | 8.3 | 8.4 |
| | 8.2 | 8.9 | | 8.3 | 8.3 | 8.4 |

**Tabelle 1b**

| pH-Wert der Konjugat-Stammlsg | pH-Wert des Tränkpuffers | pH-Wert nach Verd. auf OD8 | OD der Konjugat-Stammlsg. | OD sofort n. Verdünnung | OD nach 1 Std. | OD nach 6 Std. |
|---|---|---|---|---|---|---|
| 8.5 | 4.3 | 6.7 | 21.49 | 8.4 | 8.4 | 8.5 |
| | 5.3 | 7.1 | | 8.5 | 8.5 | 8.6 |
| | 7.0 | 7.6 | | 8.4 | 8.4 | 8.5 |
| | 7.5 | 8.2 | | 8.5 | 8.6 | 8.6 |
| | 8.2 | 8.9 | | 8.3 | 8.2 | 8.5 |

**Tabelle 1c**

| pH-Wert der Konjugat-Stammlsg | pH-Wert des Tränkpuffers | pH-Wert nach Verd. auf ODB | OD der Konjugat-Stammlsg. | OD sofort n. Verdünnung | OD nach 1 Std. | OD nach 6 Std. |
|---|---|---|---|---|---|---|
| 8.6 | 4.5 | 6.6 | 20.20 | 8.1 | 6.4 | 5.2 |
| | 4.8 | 7.4 | | 7.8 | 6.4 | 6.2 |
| | 5.2 | 7.5 | | 7.9 | 7.3 | 7.3 |
| | 6.0 | 7.8 | | 8.1 | 8.0 | 8.1 |
| | 7.5 | 8.0 | | 7.9 | 8.0 | 8.1 |
| | 8.8 | 8.6 | | 8.0 | 8.0 | 8.2 |

Diese Ergebnisse zeigen, daß beim Goldkonjugat des Standes der Technik bei pH-Werten im sauren und neutralen Bereich bereits nach 1 Stunde eine deutliche Instabilität (Abnahme der OD) zu beobachten ist. Die OD der erfindungsgemäßen Konjugate bleibt hingegen konstant. Dies zeigt, daß die erfindungsgemäßen Konjugate eine deutlich verbesserte pH-Stabilität als die Konjugate des Standes der Technik besitzen.

### Beispiel 7

### Vergleich der Testfunktion von erfindungsgemäßen Antikörper-Goldkonjugaten mit Konjugaten des Standes der Technik

Es wurde ein Konjugat aus einem polyklonalen Anti-Amphetamin-Schaf-IgG-Antikörper und 20 nm Goldpartikeln hergestellt. Die Nachsättigung erfolgte durch Zusatz von 0,1 % RSA. Der Lagerpuffer enthielt 2 % RSA.

Zur erfindungsgemäßen Stabilisierung der Konjugate erfolgte eine Zugabe von 5·10⁻⁶ M oder 10⁻⁵ M PEG-SH nach der Nachsättigung oder zum Lagerpuffer.

Der Amphetaminnachweis wurde als kompetitiver Ein-Schritt-Test auf einem Teststreifen durchgeführt.

Das Ergebnis dieses Versuchs ist in Abb. 1 gezeigt. Dort ist zu erkennen, daß das Goldkonjugat ohne Zusatz von PEG-SH eine deutlich flachere Testkurve und somit eine schlechtere Testfunktion zeigt als die erfindungsgemäßen, durch PEG-SH stabilisierten Konjugate. Ein Zusatz von 10⁻⁵ M PEG-SH führt zu einer noch stärkeren Verbesserung als der Zusatz von 5·10⁻⁶ M PEG-SH.

## Patentansprüche

1. Zusammensetzung, umfassend kolloidale Partikel, an deren Oberfläche Biomoleküle adsorbiert sind,
**dadurch gekennzeichnet, daß** die Zusammensetzung weiterhin ein durch Thioloder/und Disulfidgruppen substituiertes Polyethylenglykol enthält.

2. Zusammensetzung nach Anspruch 1,
**dadurch gekennzeichnet, daß** das substituierte Polyethylenglykol an der Oberfläche der Partikel coadsorbiert ist.

3. Zusammensetzung nach Anspruch 1 oder 2,
**dadurch gekennzeichnet, daß** die Partikel Edelmetallpartikel sind.

4. Zusammensetzung nach einem der Ansprüche 1-3,
**dadurch gekennzeichnet, daß** der mittlere Durchmesser der Partikel im Bereich von 1 nm bis 1000 nm ist.

5. Zusammensetzung nach einem der Ansprüche 1-4,
**dadurch gekennzeichnet, daß** die Biomoleküle ausgewählt sind aus der Gruppe bestehend aus Proteinen, Glycoproteinen, Peptiden, Nucleinsäuren, peptidischen Nucleinsäuren, Sacchariden, Antigenen und Haptenen.

6. Zusammensetzung nach Anspruch 5,
**dadurch gekennzeichnet, daß** die Biomoleküle aus der Gruppe bestehend aus Antikörpern, Antikörperfragmenten, Lectinen, Enzymen, Streptavidin, Avidin, Protein A, rekombinanten Polypeptiden, Peptiden, Haptenen und Polyhaptenen ausgewählt sind.

7. Zusammensetzung nach einem der Ansprüche 1-6,
**dadurch gekennzeichnet, daß** das substituierte Polyethylenglykol die allgemeine Strukturformel
HS - R¹ (I)
oder 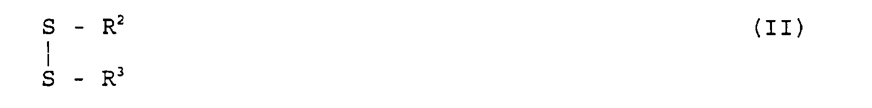 aufweist, worin der Rest R¹ sowie mindestens einer der Reste R² und R³ ein organischer Rest ist, der mindestens 2 und vorzugsweise mindestens 3 (CH₂-CH₂-O)-Gruppen enthält.

8. Zusammensetzung nach einem der Ansprüche 1-7,
**dadurch gekennzeichnet, daß** das substituierte Polyethylenglykol ein maximales mittleres Molekulargewicht von etwa 50 000 D aufweist.

9. Zusammensetzung nach Anspruch 7,
**dadurch gekennzeichnet, daß** die Reste R¹, R² und R³ als Endgruppen OH-Gruppen oder/und O-Alkyl-Gruppen enthalten.

10. Verwendung einer Zusammensetzung nach einem der Ansprüche 1-9 als Nachweisreagenz.

11. Verwendung nach Anspruch 10 zur Anfärbung von Gewebeschnitten.

12. Verfahren zur Stabilisierung von Konjugaten aus kolloidalen Metallpartikeln und Biomolekülen,
**dadurch gekennzeichnet, daß** man dem Konjugat ein durch Thiol- oder/und Disulfidgruppen substituiertes Polyethylenglykol zusetzt.

13. Verfahren nach Anspruch 12,
**dadurch gekennzeichnet, daß** man das substituierte Polyethylenglykol in einer Menge zusetzt, um eine Endkonzentration von 0,1 µM bis 1 mM zu ergeben.

14. Verfahren nach Anspruch 12 oder 13,
**dadurch gekennzeichnet, daß** man weiterhin einen zusätzlichen Stabilisator zusetzt.

15. Testkit für ein immunologisches Nachweisverfahren,
**dadurch gekennzeichnet, daß** er eine stabilisierte Zusammensetzung nach einem der Ansprüche 1-9 als Nachweisreagenz enthält.

## Claims

1. A composition, comprising colloidal particles, on the surface of which biomolecules are adsorbed, **characterised in that** the composition also contains a polyethylene glycol substituted by thiol and/or disulphide groups.

2. A composition according to claim 1, **characterised in that** the substituted polyethylene glycol is co-adsorbed on the surface of the particles.

3. A composition according to claim 1 or 2, **characterised in that** the particles are precious metal particles.

4. A composition according to any one of claims 1 to 3, **characterised in that** the mean diameter of the particles is in the range of 1 nm to 1,000 nm.

5. A composition according to any one of claims 1 to 4, **characterised in that** the biomolecules are selected from the group consisting of proteins, glycoproteins, peptides, nucleic acids, peptidic nucleic acids, saccharides, antigens and haptens.

6. A composition according to claim 5, **characterised in that** the biomolecules are selected from the group consisting of antibodies, antibody fragments, lectins, enzymes, streptavidin, avidin, protein A, recombinant polypeptides, peptides, haptens and polyhaptens.

7. A composition according to any one of claims 1 to 6, **characterised in that** the substituted polyethylene glycol has the general structure formula
HS - R¹ (I)
or 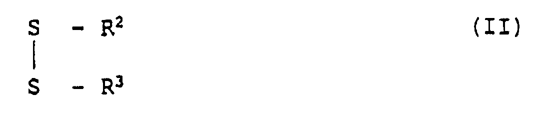 wherein the radical R¹ and at least one of the radicals R² and R³ is an organic radical containing at least 2 and preferably at least 3 (CH₂-CH₂-O) groups.

8. A composition according to any one of claims 1 to 7, **characterised in that** the substituted polyethylene glycol has a maximum mean molecular weight of about 50,000 D.

9. A composition according to claim 7, **characterised in that** the radicals R¹, R² and R³ contain OH groups or/and O-alkyl groups as terminal groups.

10. Use of a composition according to any one of claims 1 to 9 as test reagent.

11. Use according to claim 10 for dying fabric cuts.

12. A method for stabilising conjugates made of colloidal metal particles and biomolecules, **characterised in that** a polyethylene glycol substituted by thiol and/or disulphide groups is added to the conjugate.

13. A method according to claim 12, **characterised in that** the substituted polyethylene glycol is added in a quantity to yield a final concentration of 0.1 µM to 1 mM.

14. A method according to claim 12 or 13, **characterised in that** an additional stabiliser is also added.

15. A test kit for an immunological test method, **characterised in that** it contains a stabilised composition according to any one of claims 1 to 9 as test reagent.

## Revendications

1. Composition comprenant des particules colloïdales à la surface desquelles sont adsorbées des biommolécules,
**caractérisée en ce que**,
la composition contient de plus un polyéthylène-glycol substitué par des groupes thiol ou/et bisulfure.

2. Composition selon la revendication 1,
**caractérisée en ce que**,
le polyéthylène-glycol substitué est co-adsorbé à la surface des particules.

3. Composition selon la revendication 1 ou 2,
**caractérisée en ce que**,
les particules sont des particules de métaux nobles.

4. Composition selon l'une des revendications 1-3,
**caractérisée en ce que**,
le diamètre moyen des particules se situe dans la plage de 1 nm à 1000 nm.

5. Composition selon l'une des revendications 1-4,
**caractérisée en ce que**,
les biomolécules sont choisies à partir du groupe constitué par des protéines, glycoprotéines, peptides, acides nucléiques, acides nucléiques peptidiques, saccharides, antigènes et haptènes.

6. Composition selon la revendication 5,
**caractérisé en ce que**,
les biomolécules sont choisies dans le groupe constitué par des anticorps, fragments d'anticorps, lectines, enzymes, streptavidine, avidine, protéine A, polypeptides recombinants, peptides, haptènes et polyhaptènes.

7. Composition selon l'une des revendications 1-6,
**caractérisé en ce que**,
le polyéthylène-glycol substitué présente la formule structurelle générale
HS - R¹ (I)
ou 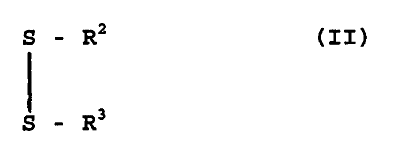 où le reste R¹ ainsi qu'au moins l'un des restes R² et R³ est un reste organique qui contient au moins 2 et de préférence au moins 3 groupes (CH₂-CH₂-O).

8. Composition selon l'une des revendications 1-7,
**caractérisée en ce que**,
le polyéthylène-glycol substitué présente un poids moléculaire moyen maximum d'environ 50 000 D.

9. Composition selon la revendication 7,
**caractérisée en ce que**,
les restes R¹,R²,R³ contiennent en tant que groupes finaux des groupes OH ou/et des groupes O-alkyle.

10. Utilisation d'une composition selon l'une des revendications 1-9 en tant que réactif indentificateur.

11. Utilisation selon la revendication 10 pour la coloration de sections de tissus.

12. Procédé pour la stabilisation de conjugués à partir de particules métalliques colloïdales et de biomolécules,
**caractérisé en ce que**,
l'on ajoute au conjugué un polyéthylène-glycol substitué par les groupes thiol ou/et bisulfure.

13. Procédé selon la revendication 12,
**caractérisé en ce que**,
l'on ajoute le polyéthylène-glycol substitué dans une quantité permettant d'obtenir une concentration finale de 0,1 µM à 1 mM.

14. Procédé selon la revendication 12 ou 13,
**caractérisé en ce que**,
l'on ajoute de plus un stabilisateur supplémentaire.

15. Kit ou trousse d'essai pour un procédé d'identification immunologique,
**caractérisé en ce que**,
il contient une composition stabilisée selon l'une des revendications 1-9 en tant que réactif identificateur.
